(19) Europäisches Patentamt
European Patent Office
Office européen des brevets

(11) EP 4 792 806 A1

(12) EUROPEAN PATENT APPLICATION

(43) Date of publication:
**19.08.2026 Bulletin 2026/34**

(21) Application number: **26155785.4**

(22) Date of filing: **02.02.2026**

(51) International Patent Classification (IPC):
***A61K 9/24*** *(2006.01)* ***A61K 9/14*** *(2006.01)*
***A61K 31/4045*** *(2006.01)* ***A61K 31/405*** *(2006.01)*

(52) Cooperative Patent Classification (CPC):
(C-Sets available)
**A61K 9/209; A61K 9/146; A61K 31/4045; A61K 31/405** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **13.02.2025 IT 202500002703**

(71) Applicant: **Inalme S.r.l.**
**95121 Catania (IT)**

(72) Inventors:
- **CICCIARELLA, Enzo**
**I-95121 CATANIA (IT)**
- **ABATE, Pietro**
**I-95121 CATANIA (IT)**
- **LOMBARDO, Rosamaria**
**I-95121 CATANIA (IT)**

(74) Representative: **Long, Giorgio**
**Jacobacci & Partners S.p.A.**
**Via Senato, 8**
**20121 Milano (IT)**

# (54) PROLONGED-RELEASE TABLET CONTAINING MELATONIN AND L-TRYPTOPHAN

(57) It forms the subject of the present invention to provide a formulation in the form of a prolonged-release tablet, namely a bilayer tablet, containing melatonin and other active ingredients.

In particular, the present invention relates to a formulation in multilayer tablet form comprising:

- a first layer containing a mixture of gastro-resistant microparticles of melatonin and gastro-resistant microparticles of L-tryptophan,
- a second layer containing a mixture of melatonin and a butyrate salt.

EP 4 792 806 A1

(52) Cooperative Patent Classification (CPC): (Cont.)

C-Sets
**A61K 31/4045, A61K 2300/00;**
**A61K 31/405, A61K 2300/00**

## Description

### Field of the invention

It forms the subject of the present invention to provide a formulation in the form of a prolonged-release tablet, namely a bilayer tablet, containing melatonin and other active ingredients.

### State of the art

Attention deficit hyperactivity disorder (ADHD) is one of the most common childhood behavioural disorders, with a prevalence of around 5% in school-age children, with early onset, usually within the first 7 years of life.

It is estimated that in at least 55% of ADHD cases, there are problems associated with sleep that are generally so disabling for both children and parents that they overshadow the severity of the classic daytime behavioural symptoms.

Moreover, the two ADHD symptoms, daytime and nighttime, seem to feed off each other as any sleep disturbance, difficulty falling asleep, inadequate duration and fragmented or interrupted sleep, may contribute to the onset or worsening of mood, attention and behavioural problems. On the other hand, a constant state of hyperactivity may hinder the nocturnal onset of sleep, thus delaying the falling asleep phase.

It follows that targeted treatment and management of sleep problems in children with ADHD could significantly reduce the severity of behavioural symptoms and improve the quality of life of both the children themselves and their families. In addition, both sleep disturbances and behavioural changes typical of ADHD are correlated with premature inflammation in the small and large intestine.

Both melatonin and tryptophan are molecules used, in low and controlled dosages, to treat sleep disorders and are often found in food supplements and dietary formulations.

However, there are two requirements that state-of-the-art formulations are unable to meet. It is indeed important that an administered composition can speed up the falling asleep phase, but also maintain the depth of NREM sleep, thus avoiding unpleasant night waking.

### Summary of the invention

The aforementioned need is addressed by the present invention, which proposes a formulation containing melatonin and tryptophan with controlled release, i.e., capable of releasing one or more active ingredients in both the gastric and the intestinal environment.

The present invention therefore relates to a multilayer tablet formulation as outlined in the annexed claims.

The subjects of the invention are in particular:

1) a multi-layer tablet formulation containing:

- a first layer containing a mixture of gastro-resistant microparticles of melatonin and gastro-resistant microparticles of L-tryptophan,
- a second layer containing a mixture of melatonin and a butyrate salt;

2) a formulation as in point 1, in which said tablet is a bilayer tablet;
3) a formulation as in 1 or 2, in which the microparticles of the first layer contain a gastro-resistant polymer;
4) a formulation as in step 3, where the gastro-resistant polymer is a copolymer of methacrylic acid and methyl methacrylate, preferably, the copolymer Eudraguard® Control;
5) a formulation as in any one of 1 to 4, in which in said microparticles the weight ratio melatonin/gastro-resistant polymer is between 1:10 and 1:20 and the weight ratio L-tryptophan/gastro-resistant polymer is between 1:10 and 1:20;
6) a formulation as in point 5, in which the melatonin/polymer weight ratio is about 1:10 and the L-tryptophan/polymer weight ratio is about 1:20;
7) a formulation as in any of points 1 to 6, in which in the gastro-resistant melatonin microparticles the amount of melatonin is between 0.125 and 0.5 mg, and in the gastro-resistant L-tryptophan particles the amount of L-tryptophan is between 5 and 20 mg;
8) a formulation as in any one of points 1 to 7, in which, in the second layer, melatonin is in free form and is contained in an amount between 0.125 and 0.5 mg;
9) a formulation as in any one of points 1 to 8, in which, in the second layer, the butyrate salt, preferably sodium butyrate, is contained in quantities between 75 and 300 mg;
10) a formulation as in any one of points 1 to 9, in which said multilayer tablet has a thickness of 8-9 mm and a hardness

of between 15 and 20 N;

(11) dietetic compositions, food supplements and foods for special medical purposes (AFMS) containing the formulation as in any of points 1 to 10;

12) A procedure for the preparation of formulations as in any one of points 1 to 10, comprising a direct compression step of a mixture of components of the first layer and a mixture of components of the second layer, in which the maximum compression force is 80 KN and the relative humidity (RH%) is 40-50%.

These and further items will be described below. The text of the claims must be considered to be included in the description for the purpose of assessing sufficiency of description.

Further features and advantages of the invention will result from the description below of preferred examples of embodiments, given by way of indication and not limitation.

## Brief description of the figures

Figure 1 shows a graph representing the Z-potential (ZP) analysis of Blank (sample without active ingredients), MEc-MPs-10 (melatonin/polymer microparticles 1:10), MEc-MPs-20 (melatonin/polymer microparticles 1:20), TEc-MPs-10 (tryptophan/polymer microparticles 1:10) and TEc-MPs-20 (tryptophan/polymer microparticles 1:20);

Figure 2 shows an FT-IR spectrum in which: (a) Melatonin; (b) Eudraguard® Control polymer (EUGC); (c) Blank; (d) MEc-MPs-10; (e) MEc-MPs-20;

Figure 3 shows an FT-IR spectrum in which: (a) L-Tryptophan; (b) EUGC; (c) Blank; (d) TEc-MPs-10; (e) TEc-MPs-20;

Figure 4 shows a DSC spectrum in which: (a) Melatonin; (b) Melatonin/EUGC 1:20 mixture; (c) Melatonin/EUGC 1:10 mixture; (d) Aerosil®-200; (e) EUGC; (f) Blank; (g) MEc-MPs-10; (h) MEc-MPs-20;

Figure 5 shows a DSC spectrum in which: (a) L-Tryptophan; (b) L-Tryptophan/EUGC 1:20 mixture; (c) L-Tryptophan/EUGC 1:10 mixture; (d) Aerosil®-200; (e) EUGC; (f) Blank; (g) TEc-MPs-10; (h) TEc-MPs-20;

Figure 6 shows the release profile of MEc-MPs-10 (bottom curve) and MEc-MPs-20 (top curve) in SGF (pH 1) for 2 h, in SIF (pH 6.8) for 6 h and in SCF (pH 7.4) for 24 h incubated at 37°C under 150 rpm stirring;

Figure 7 shows the release profile of TEc-MPs-10 and TEc-MPs-20 in SGF (pH 1) for 2 h, in SIF (pH 6.8) for 6 h and in SCF (pH 7.4) for 24 h incubated at 37°C under stirring at 150 rpm.

## Detailed description of the invention

The present invention relates to a formulation in multilayer tablet form containing:

- a first layer containing a mixture of gastro-resistant microparticles of melatonin and gastro-resistant microparticles of L-tryptophan;
- a second layer containing a mixture of melatonin and a butyrate salt.

The microparticles of the first layer comprise a gastro-resistant polymer in which, preferably, the weight ratio of active ingredient to polymer is between 1:10 and 1:20.

In preferred embodiments, the melatonin/polymer weight ratio is about 1:10 and the L-tryptophan/polymer weight ratio is about 1:20.

In preferred embodiments, the gastro-resistant polymer is a copolymer of methacrylic acid and methyl methacrylate. More preferably, the Eudraguard® Control copolymer (EUGC) marketed by Evonik will be used.

EUGC is a recognised safe food grade copolymer (GRAS) and has been selected for the purposes of the present invention mainly because of two of its characteristics: (i) gastro-resistance, to protect the encapsulated active ingredients from the acidic pH of the stomach and environmental factors during storage; (ii) controlled, slow, targeted and pH-dependent release mechanism (at pH $\geq$ 6.8 - gut environment).

The polymer (EUGC) was used as a microcarrier to incorporate two compounds of nutraceutical interest (AI), melatonin (MEL) and L-tryptophan (TRP), both involved in the regulation of the sleep-wake cycle.

In melatonin microparticles, the amount of melatonin is preferably between 0.125 and 0.5 mg, while the amount of L-tryptophan in L-tryptophan microparticles is preferably between 5 and 20 mg.

In the second layer, melatonin is in free form and is present in an amount preferably between 0.125 and 0.5 mg.

In the second layer, butyrate salt, preferably sodium butyrate, is preferably present in amounts between 75 and 300 mg.

Taking into account that the permitted or recommended daily dosage of the active ingredients, for a paediatric subject, is 1 mg for melatonin, 20 mg for L-tryptophan and 300 mg for sodium butyrate, depending on the amount of active

ingredient contained in the tablets of the invention, one to four daily administrations may be envisaged.

It has been seen that the active ingredients in the above dosages and formulation profiles act synergistically.

L-tryptophan (TRP) microparticles protect the active ingredient from the action of gastric juices, then adhere to the intestinal mucosa and release TRP in a slow and controlled manner. Subsequently, of the portion of TRP released (approx. 35-45% 2 hours after administration): i) 1% reaches the CNS (Central Nervous System) to be converted into serotonin, regulating circadian rhythms and contributing to the functioning of the monoaminergic system, for proper behaviour management. Furthermore, the serotonin obtained acts as a precursor to melatonin (MEL), for the correct regulation of sleep-wake rhythms, increasing and maintaining the depth (stage IV) of NREM sleep, ii) 96% remains in the intestine to be converted into serotonin, which also triggers the synthesis of occludins, molecules capable of strengthening the *tight junctions* between one enterocyte and another, thus hindering inflammatory intestinal patterns (IBS and Leaky Gut), related to behavioural disorders, including altered sleep-wake rhythms.

The free portion of melatonin (MEL), on the other hand, is released from the tablet when it reaches the stomach and, being naturally resistant to gastric juices, reaches the small intestine to be 100% pre-absorbed. 15% of MEL is then absorbed and distributed, reaching the CNS and overcoming the BEE (Blood-Encephalic Barrier), so that the falling asleep phase is triggered within 20 min after oral administration.

Melatonin microparticles, on the other hand, prevent the release of the active ingredient in the stomach, and then adhere to the intestinal mucosa and release melatonin in a slow and controlled manner. Of the portion of MEL released by the microparticles (approximately 25-50% 2h after administration), 15% slowly reaches the CNS and maintains sleep for longer, so as to avoid early nocturnal awakenings and hinder the onset of parasomnias. In addition, during enteric internalisation, part of MEL interacts with the enteric serotonergic system, contributing to the maintenance of intestinal serotonergic activity and further enhancing the proper functioning of the gut-brain axis, which is linked to behavioural disorders, including sleep-wake rhythm alteration.

Free butyrate salt has important functions in various districts. At the intestinal level, it contributes to maintaining the integrity of the intestinal epithelial barrier, while at the central level, butyrate appears to maintain BEE integrity and induce NREM sleep.

A further object of the invention are also dietary compositions, food supplements and foods for special medical purposes (AFMS) comprising the formulation of the invention.

The term "food for special medical purposes" refers to products authorised according to Regulation (EU) 2016/128. This term refers to a product to be administered under medical supervision, thus likening this AFMS to a drug.

Formulations according to the invention can be prepared according to conventional methods, such as those described in Remington's Pharmaceutical Sciences Handbook, Mack Pub. Co., N.Y., USA, 17th edition, 1985 or in Remington, The Science and Practice of Pharmacy , Edited by Allen, Loyd V., Jr, 22nd edition, 2012 or later editions.

In preferred pharmaceutical forms, the multilayer tablet is a bilayer tablet as defined above, comprising a first layer comprising a mixture of gastro-resistant microparticles of melatonin and gastro-resistant microparticles of L-tryptophan and a second layer comprising a mixture of melatonin and a butyrate salt.

The multilayer tablet layers of the invention will typically contain, in addition to the aforementioned active ingredients, pharmaceutically acceptable excipients such as binding agents (e.g. pregelatinised maize starch, polyvinylpyrrolidone or hydroxypropyl methylcellulose); filling agents (e.g. lactose, microcrystalline cellulose or calcium hydrogen phosphate); lubricants (e.g. magnesium stearate, talc or silica); disintegrating agents (e.g. potato starch or sodium glycolate starch); or inhibiting agents (e.g. sodium lauryl sulphate).

The tablets according to the invention can be prepared according to conventional methods, such as those described in Remington's Pharmaceutical Sciences Handbook, Mack Pub. Co., N.Y., USA, 17th edition, 1985 or in Remington, The Science and Practice of Pharmacy, Edited by Allen, Loyd V., Jr, 22nd edition, 2012 or later editions.

In preferred embodiments, the multilayer tablet of the invention can be prepared by a conventional direct compression process of a mixture of components of the first layer and a mixture of components of the second layer, in which the maximum compression force is 80 KN and the relative humidity (RH%) is 40-50%.

The resulting tablets will preferably have a thickness of 8-9 mm and a hardness of between 15 and 20 N.

EXPERIMENTAL PART

The microparticles (MPs) obtained were prepared at two different ratios of active ingredient (AI) to polymer (EUGC), 1:10 (melatonin, MEc-MPs-10; tryptophan, TEc-MPs-10) and 1:20 (melatonin, MEc-MPs-20; tryptophan, TEc-MPs-20), respectively, using Aerosil®-200 (silicon dioxide) as anti-caking agent.

The MPs obtained were lyophilised and then characterised taking into account:

- the yield (Yield %),
- the particle size (Size %),
- the % of loading of active ingredients (%DL) and % encapsulation efficiency (%EE),

- the stability of loaded MPs (11 months).

To support these data, instrumental measurements were performed using:

- calculation of the zeta potential (ZP),
- FT-IR spectroscopy,
- DSC differential scanning calorimetry
- X-ray diffractometry (XRPD),
- high-performance liquid chromatography (HPLC).

*In vitro* studies then analysed the pH-dependent release rate of melatonin (MEL) and tryptophan (TRP) from MPs and assessed their potential interaction with mucin in simulated gastric fluid (SGF) and simulated intestinal fluid, both from the small intestine (SIF) and large intestine (SCF), by performing mucoadhesive studies of MPs, using turbidimetry-UV.

Eudraguard® Control (EUGC) is a food-grade neutral copolymer based on ethyl methacrylate and methyl methacrylate (2:1 molar ratio), manufactured by Evonik Industries AG (Germany) and supplied by Rofarma Italia srl (Italy), as well as 'generally recognised as safe' (GRAS) in the US and authorised in the EU (code E1205) for nutraceutical applications. It is recommended as a food biomaterial for the coating of solid pharmaceutical forms and the formation of MPs microparticles, which can mask the taste and odour of the incorporated actives (AIs) and give them discreet protection against light, moisture and atmospheric oxygen. EUGC then exhibits pH-dependent degradation, resisting degradation at pH values below 6.8, thus providing effective resistance to gastric fluid, followed by sustained release of the charged AI at pH values above 6.8 and thus in the intestine.

Preparation of microparticles (MPs)

Microparticles with the active ingredient were prepared according to conventional methods by solvent evaporation.

10 mg EUGC was mixed at 40°C under stirring (500 rpm) with 10 mg MEL or TRP in 30 ml or 40 ml ethanol to obtain MPs with an active-polymer ratio of 1:10 (MEc-MPs-10 and TEc-MPs-10) and 1:20 (MEc-MPs-20 and TEc-MPs-20), respectively.

Using a separating funnel, the resulting ethanolic solution is added drop by drop to a portion (30 ml or 40 ml) of distilled water at room temperature and under stirring (500 rpm), adding Aerosil®-200 as an anti-caking agent.

Finally, the newly obtained solution is evaporated using Rotavapor at a temperature of 37°C until a suspension of MPs is obtained.

The resulting MPs suspensions were frozen at -80°C for 24 hours and then freeze-dried at a pressure of 0.3 mbar for 48 hours, using a Buchi Lyovapor L-200 freeze-dryer.

The % yield was calculated using the formula:

**Yield % = (Final product weight (mg)/Theoretical weight: polymer + AI + silica (mg)) × 100**

The lyophilised powder was passed over sieves of 90 μm and 500 μm, respectively, and then weighed to calculate the particle size distribution %, using the formula:

$$\text{Size \%} = (W_R\ (mg) / W_I\ (mg)) \times 100$$

whereas:

- **$W_R$:** powder retained by sieves;
- **$W_I$:** initial powder.

**Instrumental analysis and characterisation**

Zeta Potential (ZP) Analysis

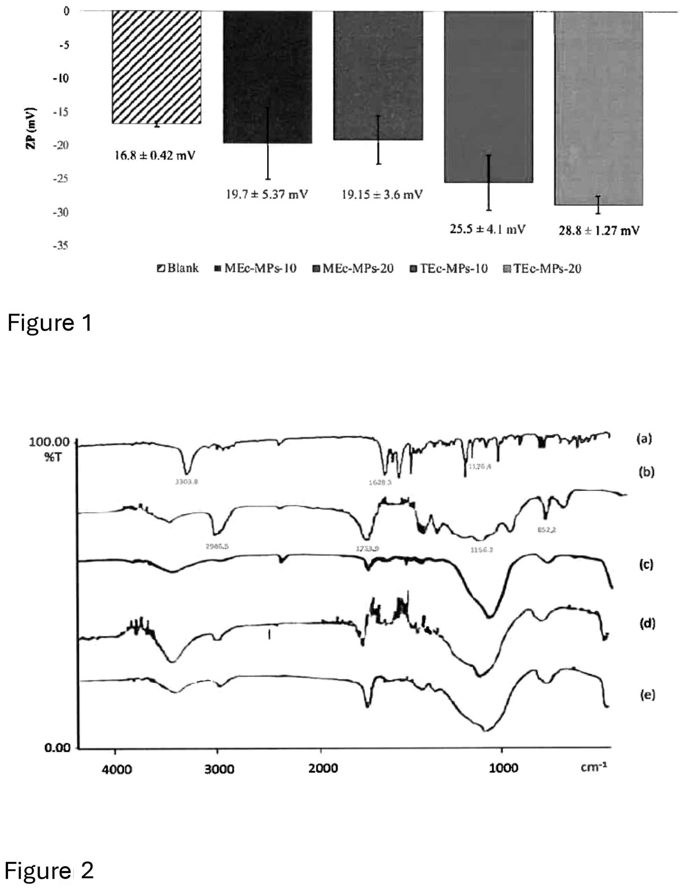

The ZP evaluation was determined to support the particle size distribution data by sieving, showing that all MPs are externally negatively charged. See table 1 and figure 1.

Furthermore, it is observed that only the MEc-MPs-10 system has MPs evenly distributed over all sieves:

Tab. 1 - Size distribution of MPs by sieving

| SAMPLES | >500 μm | 500 μm<x<90 μm | < 90 μm |
|---|---|---|---|
| MEc-MPS-10 | 35% | 28% | 36% |
| MEc-MPS-20 | 31% | 52% | 11% |
| TEc-MPS-10 | 10% | 50% | 33% |
| TEc-MPS-20 | 22% | 30% | 32% |

FT-IR spectrophotometry

FT-IR spectrophotometry made it possible to analyse possible interactions between Al and EUGC during the formation of MPs by analysing the IR spectra of both the individual raw materials (MEL, TRP and EUGC) and the unloaded (empty) MPs and their loaded systems (MEc-MPs-10, TEc-MPs-10, MEc-MPs-20 and TEc-MPs -20).

The spectrum in Figure 2 shows that, during the loading of MEL into EUGC, there was no structural change in the characteristic groups of the individual components, thus maintaining their innate characteristics.

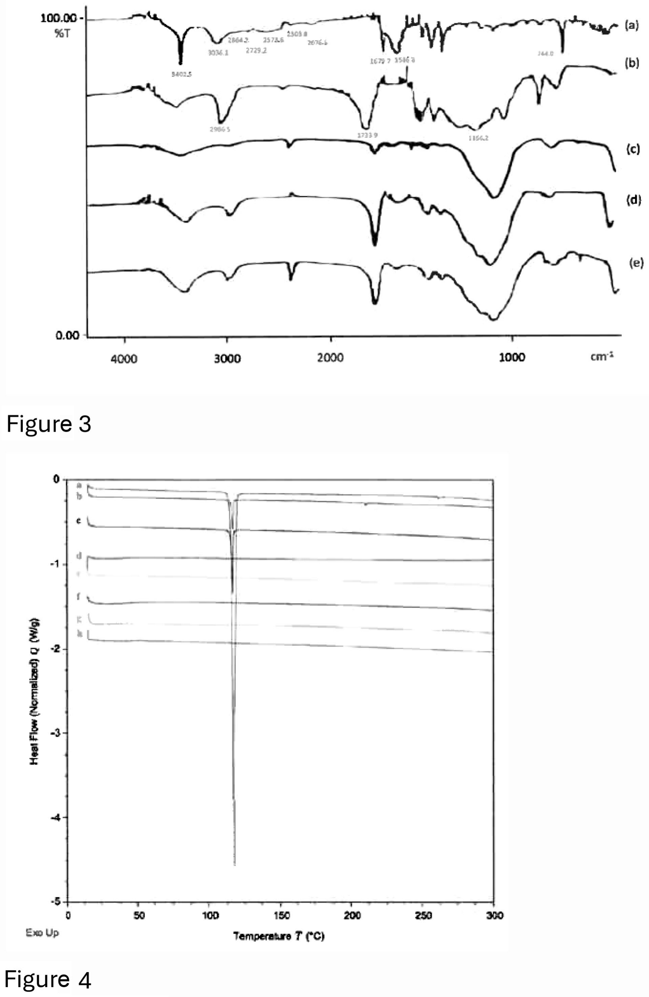

Similarly, the spectrophotometry in Figure 3 shows that, during TRP loading in EUGC, there was no structural change in the characteristic groups of the individual components, thus maintaining their innate characteristics.

Differential scanning calorimetric analysis (DSC) and X-ray powder diffraction study (XRPD)

The combination of the two techniques (DSC and XRPD) made it possible both to confirm the successful loading of Al actives into MPs and to demonstrate the absence of structural changes during their loading. Furthermore, during the loading phase, the actives underwent dispersion, charging amorphously and non-crystalline within the EUGC carrier, thus creating somewhat stable systems.

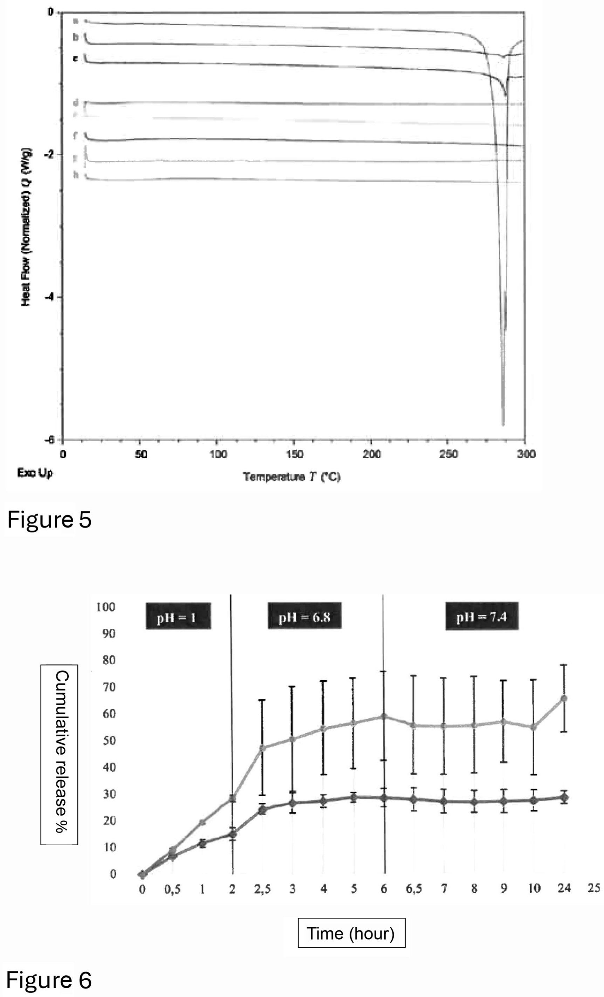

The calorimetric curves of pure MEL (Fig. 4) and pure TRP (Fig. 5) show a strong endothermic peak at 117.34°C and 286.10°C respectively, which correspond to the respective melting points (Tm) of MEL and TRP, underlining their crystalline nature. The same peaks are found in the mixtures before loading, albeit with less intensity, as they are proportional to the amount of Al present in the mixture, still in the crystalline state. In contrast, the aforementioned peaks are absent in the loaded systems, demonstrating that the Al has been encapsulated in an amorphous and no longer crystalline state.

Similar results were obtained by comparing the XRPD spectra (not shown).

The analyses also underlined a stability of the loaded systems (MPs) maintained for as long as 11 months.

Percentage loading (%DL) and encapsulation efficiency (%EE)

The loaded MPs were emptied of their respective actives (MEL and TRP), using both the vortex precipitation and ultrasonication methods. After a centrifugation step and HPLC-UV analysis, the % loading (%DL), i.e. the amount of drug loaded per unit weight of the MP, was initially quantified, indicating the percentage of the MP mass that is due to the encapsulated active. The %DL is calculated using the formula:

$$\%\ DL = \frac{\text{Current drug quantity (mg)}}{\text{Quantity of system (mg)}} \times 100$$

Subsequently, the encapsulation efficiency (%EE) is calculated, i.e. the percentage of the active ingredient that is successfully trapped in the MP. The %EE is calculated using the formula:

$$\%\ EE = \frac{\text{Current drug quantity (mg)}}{\text{Theoretical drug quantity (mg)}} \times 100$$

Both extraction methods (precipitation and sonication) showed the same results, the averages of which are shown in the table below:

Tab. 2. %DL and %EE of MEc -MP and TEc -MP

| SAMPLES | %DL ±SD | %EE ± SD |
|---|---|---|
| **ME c -MPS-10** | 7.23 ± 1.03 % | 81.70 ± 11.38 % |
| **ME c -MPS-20** | 4.36 ± 0.20 % | 91.69 ± 4.35 % |
| **TE c- MPS-10** | 3.13 ± 0.13 % | 65.48 ± 2.79 |
| **TE c -MPS-20** | 2.46 ± 0.23 | 99.71 ± 9.49 |

Release profile - *In vitro* studies

The in vitro pH-dependent release profile of MEL and TRP was conducted using the 'sample and separation' (SS) method. The test was performed for 24 hours at 37°C under stirring (150 rpm) in three different artificial media, reproducing the entire gastrointestinal (GI) environment and respectively:

- the simulated gastric fluid (SGF) at pH 1.0, for the first 2 hours,
- simulated intestinal fluid (SIF) at pH 6.8 for the next 4 hours,
- simulated colon fluid (SCF) at pH 7.4, until the end of 24 hours.

In particular, analysing the release profile for Melatonin (MEL). the % release shown in Figure 6 are observed, i.e:

**Stomach (SGF):** 15% (MEL/EUGC 1:10) and 25% (MEL/EUGC 1:20) after 2 h
**Small intestine (SIF):** 25%(MEL/EUGC 1:10) and 50%(MEL/EUGC 1:20) after 30 min (2.5 h)
**Large intestine (SCF):** plateau + 70%(MEL/EUGC 1:20) after 24 h.

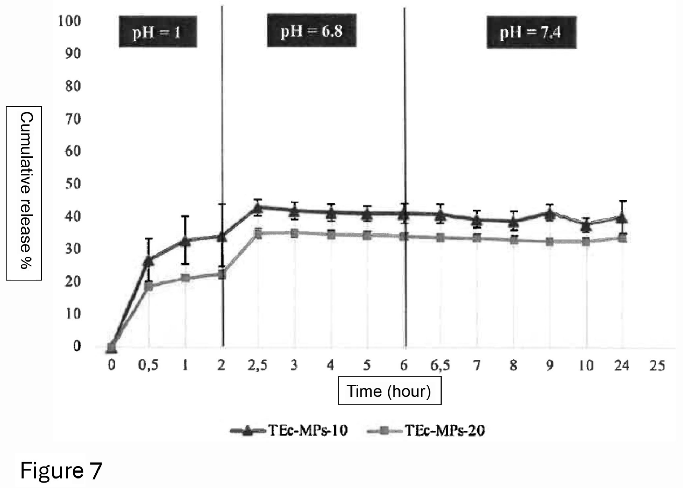

A spike in the released portion of MEL is also observed 30 min after the arrival of MPs in the small intestine (SIF).
Analysing the release profile for tryptophan (TRP) instead, we observe the release % shown in Figure 7, i.e:

**Stomach (SGF):** 20% (TRP/EUGC 1:10) and 34% (TRP/EUGC 1:20) after 2 h,
**Small intestine (SIF):** 35%(TRP/EUGC 1:10) and 45%(TRP/EUGC 1:20) after 30 min (2.5 h)
**Large intestine (SCF):** plateau after 24 h

Once again, a spike in the released portion of TRP is observed 30 min after the arrival of MPs in the small intestine (SIF).

Mucoadhesive study

The evaluation of the potential mucoadhesive properties of MPs was carried out by means of a turbidimetric assay, using artificial mucin and a UV spectrophotometer (A = 650 nm). Turbidity values were calculated according to the following formula:

$$\Delta abs = A - A_{theor}$$

whereas:

- **A:** effective absorbance of MP-mucine mixtures
- $A_{theor}$: theoretical absorbance

The degree of turbidity, proportional to the degree of mucoadhesiveness and calculated as the difference (Δabs), was taken as a reference measure of the interaction between mucin and MPs. In particular:

- **Δabs ≤ 0:** partial or no mucoadhesiveness,
- **Δabs > 0:** intense mucoadhesiveness.

Measurements were performed in three different media, in **SGF (pH 1),** to simulate pre-prandial gastric conditions, in **SGF (pH 3), to simulate post-prandial gastric conditions, and in SIF** at pH 6.8 to simulate the final intestinal stages of digestion.
The following values are observed:

Change in Δabs in blank, MEc-MPs-10, MEc-MPs-20, TEc-MPs-10, TEc-MPs-20 mixed with mucin 0.1% (w/v) and incubated at 37°C in SGF pH 1 for 0, 0.5, 1, 1.5 and 2 h

| | 0 | 0.5 | 1 | 1.5 | 2 |
|---|---|---|---|---|---|
| Blank mucin | -0.247 | -0.222 | -0.216 | -0.199 | -0.199 |
| MEC-MPs-10-mucin | -0.231 | -0.208 | -0.208 | -0.194 | -0.066 |
| MEC-MPs-20-mucin | -0.23 | -0.2 | -0.177 | -0.174 | -0.162 |
| TEC-MPs-10-mucin | -0.289 | -0.23 | -0.204 | -0.202 | -0.206 |
| TEC-MPs-20-mucin | -0.248 | -0.224 | -0.223 | -0.213 | -0.248 |

Under **pre-prandial conditions** (SGF pH 1) Δabs <0 denotes a very low mucoadhesiveness that nevertheless increases during the first 2 hours after administration, with a tendency towards Δabs = 0.

Change in Δabs in blank, MEc-MPs-10, MEc-MPs-20, TEc-MPs-10, TEc-MPs-20 mixed with mucin 0.1% (w/v) and incubated at 37°C in SGF pH 3 for 0, 0.5, 1, 1.5 and 2 h

| | 0 | 0.5 | 1 | 1.5 | 2 |
|---|---|---|---|---|---|
| Blank mucin | -0.11 | -0.11 | -0.089 | -0.06 | -0.0675 |
| MEC-MPs-10-mucin | -0.301 | -0.309 | -0.301 | -0.289 | -0.233 |
| MEC-MPs-20-mucin | -0.305 | -0.263 | -0.279 | -0.281 | -0.234 |
| TEC-MPs-10-mucin | -0.364 | -0.288 | -0.268 | -0.236 | -0.274 |
| TEC-MPs-20-mucin | -0.29 | -0.314 | -0.329 | -0.313 | -0.334 |

Under **postprandial conditions** (SGF pH 3), the same trend as above is observed, again denoting very poor mucoadhesiveness.

Change in Δabs in blank, MEc-MPs-10, MEc-MPs-20, TEc-MPs-10, TEc-MPs-20 mixed with mucin 0.1% (w/v) and incubated at 37°C in SIF at pH 6.8 for 0, 0.5, 1, 2, 3, 4, 5, 6 and 24 h

| | 0 | 0.5 | 1 | 2 | 3 | 4 | 5 | 6 | 24 |
|---|---|---|---|---|---|---|---|---|---|
| Blank mucin | -0.73 | -0.1075 | -0.113 | -0.101 | -0.178 | -0.135 | -0.178 | -0.1365 | -0.0845 |
| MEC-MPs-10-mucin | -0.088 | -0.0845 | -0.0925 | -0.0765 | -0.056 | -0.0565 | -0.064 | -0.004 | 0.14 |
| MEC-MPs-20-mucin | -0.105 | -0.0895 | -0.0745 | -0.061 | -0.1105 | -0.1155 | -0.0935 | -0.0255 | 0, .27 |
| TEC-MPs-10-mucin | -0.087 | -0.0625 | -0.0595 | -0.054 | -0.07 | -0.114 | -0.088 | -0.043 | 0.111 |
| TEC-MPs-20-mucin | -0.1225 | -0.125 | -0.111 | -0.121 | -0.098 | -0.104 | -0.092 | -0.09 | 0.1245 |

In contrast, during **intestinal digestion** (SIF at pH 6.8) from 6 hours of incubation onwards, MEc-MPs-10, MEc-MPs-20 and TEc-MPs-10 already show a Δabs tending towards neutrality **(Δabs = 0).** Furthermore, after 24 hours of incubation, all four systems (MEc-MPs-10, MEc-MPs-20, TEc-MPs-10 and TEc-MPs-20), but mainly MEc-MPs-10 and TEc-MPs-20, showed a strong interaction with mucin and thus a high mucoadhesiveness **(Δabs > 0).**

From the analyses carried out and the results obtained, it can be stated that:
The production process used for the synthesis of MPs resulted in high yields (85-95%),

- The MPs mainly obtained are larger than 500 μm and smaller than 90 μm, and have a negative net charge,
- The formulation process did not cause any alterations to the chemical-physical characteristics of the polymer used (EUGC) or the charged actives (Als) (MEL and TRP),
- the stability of MPs systems has been confirmed up to at least 11 months,
- active ingredients (Als) have been distributed in the polymer matrix of the MPs in dispersed form, encapsulating themselves in an amorphous state,
- %EE was higher in systems with an active-polymer ratio of 1:20 compared to 1:10,
- all systems showed a sustained release profile,

- EUGC-based MPs showed potential gastro-resistant properties. AI release was indeed lower in SGF (pH 1), compared to SIF (pH 6.8) and SCF (pH 7.4). Furthermore, when the pH increased from 1 to 6.8, the release curves of all systems showed a spike in release over 30 minutes, indicating that in SIF the polymer chains interact more with the solvent molecules, increasing the porosity of the polymer matrix and thus the release of AIs,
- the described release profiles and gastro-resistance properties of the polymer are more evident in MEc-MPs-10 and TEc-MPs-20 than in MEc-MPs-20 and TEc-MPs-10,
- all systems showed no potential mucoadhesive properties in SGF (pH 1-3). This can certainly be helpful in favouring the transit of MPs in the intestinal lumen, where, as a result of the interactions with mucin shown in SIF at pH 6.8 after 24 hours of incubation, they could reside for a longer time thus facilitating AI absorption,
- the best systems in terms of both release and mucoadhesiveness are MEc-MPs-10 and TEc-MPs-20.

EXAMPLE OF TABLET OF THE INVENTION

Tablet weight: 1000 to 1300 mg

| Ingredient | Per 1000/1300mg | Activities | |
|---|---|---|---|
| **First layer (controlled release)** | | | |
| Microencapsulated melatonin (Eudraguard) | 3.45 mg | Active ingredient | |
| Of which melatonin | 0.25 mg | | |
| Microencapsulated tryptophan (Eudraguard) | **406 mg** | Active ingredient | |
| Of which tryptophan | 10mg | | |
| Silicon dioxide | **20mg to 26mg** | Anti-caking additive | |
| Magnesium stearate | **10 mg to 13 mg** | Sliding agent additive | |
| Microcrystalline cellulose | **From 60.55mg to 201.55mg** | Cohesion additive | |
| **Second layer (immediate release)** | | | |
| Melatonin | **0.25 mg** | Active ingredient | |
| Sodium butyrate | **150mg** | Active ingredient | |
| Silicon dioxide | **20 mg to 26 mg** | Anti-caking additive | |
| Magnesium stearate | **10 mg to 13 mg** | Sliding agent additive | |
| Microcrystalline cellulose | **319.75 mg to 460.75 mg** | Cohesion additive | |

**Production parameters:**

Machinery: B&D PZ 1500 compressor
Machine setting: Bilayer
Average production speed:30,000 tablets/hour
Max. compression pressure:80 KN
Max. pre-compression pressure: 40 KN
Turret speed:60 rpm

**Environmental production conditions:**

Temperature:20-25°C
Relative humidity (RH%):40-50 %
Pressure:1 atm.

## Claims

1. Multi-layer tablet formulation containing:

   - a first layer containing a mixture of gastro-resistant microparticles of melatonin and gastro-resistant microparticles of L-tryptophan;
   - a second layer containing a mixture of melatonin and a butyrate salt.

2. Formulation according to claim 1, wherein said tablet is a bilayer tablet.

3. Formulation according to claim 1 or 2, in which the microparticles of the first layer comprise a gastro-resistant polymer.

4. Formulation according to claim 3, wherein the gastro-resistant polymer is a copolymer of methacrylic acid and methyl methacrylate, preferably, the copolymer Eudraguard® Control.

5. Formulation according to any one of claims 1 to 4, wherein in said microparticles the weight ratio melatonin/gastro-resistant polymer is between 1:10 and 1:20 and the weight ratio L-tryptophan/gastro-resistant polymer is between 1:10 and 1:20.

6. Formulation according to claim 5, in which the melatonin/polymer weight ratio is approximately 1:10 and the L-tryptophan/polymer weight ratio is approximately 1:20.

7. Formulation according to any one of claims 1 to 6, where in the gastro-resistant melatonin microparticles the amount of melatonin is between 0.125 and 0.5 mg, and in the gastro-resistant L-tryptophan microparticles the amount of L-tryptophan is between 5 and 20 mg.

8. Formulation according to any one of claims 1 to 7, in which, in the second layer, melatonin is in free form and is contained in an amount between 0.125 and 0.5 mg.

9. Formulation according to any one of claims 1 to 8, in which, in the second layer, butyrate salt, preferably sodium butyrate, is contained in quantities between 75 and 300 mg.

10. Formulation according to any one of claims 1 to 9, in which the multilayer tablet has a thickness of 8 to 9 mm and a hardness of between 15 and 20 N.

11. Dietetic compositions, food supplements and foods for special medical purposes (AFMS) containing the formulation according to any one of claims 1 to 10.

12. A process for preparing formulations according to any one of claims 1 to 10, comprising a direct compression step of a mixture of components of the first layer and a mixture of components of the second layer, wherein the maximum compression force is 80 KN and the relative humidity (RH%) is 40-50%.

0
-5
-10
-15
-20
-25
-30
-35
ZP (mV)
16.8 ± 0.42 mV
19.7 ± 5.37 mV
19.15 ± 3.6 mV
25.5 ± 4.1 mV
28.8 ± 1.27 mV
Blank
MEc-MPs-10
MEc-MPs-20
TEc-MPs-10
TEc-MPs-20

Figure 1

100.00
%T
(a)
(b)
(c)
(d)
(e)
0.00
4000
3000
2000
1000
cm-1

Figure 2

100.00
%T
0.00
4000
3000
2000
1000
cm-1
(a)
(b)
(c)
(d)
(e)
3402.5
3036.1
2864.2
2729.2
2578.6
2986.5
1733.9
1156.2

Figure 3

0
-1
-2
-3
-4
-5
Heat Flow (Normalized) Q (W/g)
a
b
c
d
f
0
50
100
150
200
250
300
Temperature T (°C)
Exo Up

Figure 4

Heat Flow (Normalized) Q (W/g)
Temperature T (°C)
Exo Up
0
-2
-4
-6
0
50
100
150
200
250
300
a
b
c
d
e
f
g
h

Figure 5

pH = 1
pH = 6.8
pH = 7.4
Cumulative release %
Time (hour)
100
90
80
70
60
50
40
30
20
10
0
0
0,5
1
2
2,5
3
4
5
6
6,5
7
8
9
10
24
25

Figure 6

pH = 1
pH = 6.8
pH = 7.4
Cumulative release %
100
90
80
70
60
50
40
30
20
10
0
0
0,5
1
2
2,5
3
4
5
6
6,5
7
8
9
10
24
25
Time (hour)
TEc-MPs-10
TEc-MPs-20

Figure 7

Europäisches
Patentamt
European
Patent Office
Office européen
des brevets

# EUROPEAN SEARCH REPORT

Application Number
EP 26 15 5785

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2008/254121 A1 (CLEMENT KEN [CA] ET AL) 16 October 2008 (2008-10-16)<br>* paragraph [0002] *<br>* paragraph [0006] - paragraph [0016] *<br>* paragraph [0020] - paragraph [0021] *<br>* paragraph [0041] - paragraph [0042]; example 4 *<br>* abstract *<br>* claims 1-15 *<br>* figures 1-2 *<br>----- | 1-12 | INV.<br>A61K9/24<br>A61K9/14<br>A61K31/4045<br>A61K31/405 |
| A | US 2023/000826 A1 (RINALDI DANNY [US] ET AL) 5 January 2023 (2023-01-05)<br>* claims 43-62 *<br>* table 7 *<br>----- | 1-12 | |
| A | EP 4 019 017 A1 (URIACH CONSUMER HEALTHCARE S L [ES])<br>29 June 2022 (2022-06-29)<br>* paragraph [0079] - paragraph [0083] *<br>* paragraph [0105]; example 1 *<br>----- | 1-12 | |
| | | | TECHNICAL FIELDS SEARCHED (IPC) |
| A | US 2019/008188 A1 (KNUTSEN LARS JACOB STRAY [US] ET AL)<br>10 January 2019 (2019-01-10)<br>* paragraph [0092] *<br>-----<br>-/-- | 1-12 | A61K |

The present search report has been drawn up for all claims

1

| Place of search | Date of completion of the search | Examiner |
|---|---|---|
| Munich | 3 May 2026 | González Carballo, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt
European
Patent Office
Office européen
des brevets

## EUROPEAN SEARCH REPORT

Application Number
EP 26 15 5785

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AHMADI SOMAYEH ET AL: "Gut microbiota in neurological diseases: Melatonin plays an important regulatory role", BIOMEDICINE & PHARMACOTHERAPY, ELSEVIER, FR, vol. 174, 21 March 2024 (2024-03-21), XP087516451, ISSN: 0753-3322, DOI: 10.1016/J.BIOPHA.2024.116487 [retrieved on 2024-03-21] * abstract * * page 4 - page 5 * * 9. Conclusion * ----- | 1-12 | |
| | | | TECHNICAL FIELDS SEARCHED (IPC) |

The present search report has been drawn up for all claims

1

| Place of search | Date of completion of the search | Examiner |
|---|---|---|
| Munich | 3 May 2026 | González Carballo, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 26 15 5785

This annex lists the patent family members relating to the patent documents cited in the above-mentioned European search report. The members are as contained in the European Patent Office EDP file on
The European Patent Office is in no way liable for these particulars which are merely given for the purpose of information.

03-05-2026

| Patent document cited in search report | Publication date | Patent family member(s) | Publication date |
|---|---|---|---|
| US 2008254121 A1 | 16-10-2008 | NONE | |
| US 2023000826 A1 | 05-01-2023 | AU 2018292531 A1 | 13-02-2020 |
| | | CA 3068577 A1 | 03-01-2019 |
| | | CN 111032059 A | 17-04-2020 |
| | | EP 3645014 A1 | 06-05-2020 |
| | | JP 2020525549 A | 27-08-2020 |
| | | US 2020138783 A1 | 07-05-2020 |
| | | US 2023000826 A1 | 05-01-2023 |
| | | US 2024415810 A1 | 19-12-2024 |
| | | WO 2019005962 A1 | 03-01-2019 |
| EP 4019017 A1 | 29-06-2022 | NONE | |
| US 2019008188 A1 | 10-01-2019 | US 2017280749 A1 | 05-10-2017 |
| | | US 2019008188 A1 | 10-01-2019 |
| | | US 2024284941 A1 | 29-08-2024 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Remington's Pharmaceutical Sciences Handbook. Mack Pub. Co., 1985
- Remington, The Science and Practice of Pharmacy. 2012